# EUROPÄISCHE PATENTSCHRIFT

(11) **EP 0 749 749 B1**
(45) Veröffentlichungstag und Bekanntmachung des Hinweises auf die Patenterteilung: **04.04.2001**
(21) Anmeldenummer: 95109787.2
(22) Anmeldetag: 23.06.1995
(51) Int. Cl.: A61K 7/48, A61K 7/00, A61K 7/50, A61K 7/06

(54) **Mehrkomponentenkonservierungsmittel für Haar- und Körperreinigungsmittel**
Multicomponents preservative composition for hair and skin cleaning composition
Système préservatif à plusieurs composés pour les compositions de nettoyage des cheveux et de la peau

(43) Veröffentlichungstag der Anmeldung: 27.12.1996
(73) Patentinhaber: DALLI-WERKE WÄSCHE- und KÖRPERPFLEGE GmbH & Co. KG, D-52220 Stolberg (DE)
(72) Erfinder: Müller, Wilfried, D-52531 Übach-Palenberg (DE); Vathje, Rainer, D-52223 Stolberg (DE)
(74) Vertreter: Sternagel, Hans-Günther, Dr.

(56) Entgegenhaltungen:
- EP-A- 0 287 805
- EP-A- 0 365 825
- EP-A- 0 570 794
- WO-A-92/21239
- DE-A- 3 837 985
- DE-A- 4 026 756

## Beschreibung

Gegenstand der Erfindung ist ein Mehrkomponentenkonservierungsmittel für Haarpflegemittel und Körperreinigungsmittel, das einen schnelleren Wirkungseintritt als bekannte Konservierungsmittel auf Basis organischer Carbonsäuren aufweist.

Kosmetische Produkte dürfen die Gesundheit des Konsumenten nicht schädigen. Diese Forderung des Gesetzgebers betrifft neben chemischen und physikalischen Aspekten auch die mikrobiologische Qualität dieser Produkte.

Trotz kontrollierter, im hygienischen Sinne einwandfreier Arbeitsweise bei der Herstellung dieser Artikel kann es beispielsweise bei der Lagerung der leeren Behältnisse, der Verschlüsse oder bei der Befüllung der Behältnisse zu nicht vorhersehbaren Problemen durch mikrobiologischen Befall (=Primärverkeimung) kommen.

Haut- und Haarreinigungsmittel werden vorzugsweise in Flaschen oder Tuben mit Schraub- oder Schnappverschluß vermarktet. Die Produktentnahme durch den Verbraucher erfolgt über Wochen hinweg in Teilmengen.

In den Behältnissen darf es zu keiner unkontrollierten Vermehrung produktschädigender oder pathogener Keime kommen. Trotz Verwendung von Behältern mit Schnappverschlüssen, mit denen sich die Behälter nach Gebrauch wieder verschließen lassen, ist es vorhersehbar und durch die Praxis bewiesen, daß Verbraucher nicht in jedem Fall die Behältnisse nach Gebrauch wieder schließen. Ungünstiger sind die Verhältnisse bei Flaschen mit Schraubverschlüssen. Die Schraubverschlüsse werden häufiger nach Gebrauch nicht wieder auf die Flaschen geschraubt. Hier kann es in stärkerem Maße zu einer Verkeimung (=Sekundärverkeimung) kommen. Besonders in Badezimmern mit warmen und feuchten klimatischen Bedingungen wird die Gefahr einer Verkeimung gesteigert.

Durch die Benutzung von verkeimten Produkten wird der Verbraucher einer nicht zu unterschätzenden gesundheitlichen Gefährdung ausgesetzt. Besonders Schleimhäute und oberflächliche Hautverletzungen sind für bestimmte Mikroorganismen besonders zugänglich und können dort zu Infektionen führen.

Auch unter ästhetischen Gesichtspunkten sind hohe Keimzahlen und längere Zeit andauernde Verkeimungen der Hautund Haarreinigungsmittel unerwünscht, selbst wenn sie keine pathogene Keime enthalten. Bestimmte nicht pathogene Keime setzen in ihrem Stoffwechselcyclus Substanzen frei, die den Geruch, die Viskosität oder Farbe der Produkte beeinflussen.

Eine wesentliche Forderung an die Sicherheit kosmetischer Mittel ist deshalb eine einwandfreie mikrobiologische Qualität der Fertigprodukte. Die Qualität der Fertigprodukte sollte beim Gebrauch nicht nur unter optimalen hygienischen Gesichtspunkten erhalten bleiben, sondern auch unter vorhersehbaren, nicht unbedingt für die Produktstabilität günstigen Bedingungen.

Durch den Einsatz von Konservierungsmittel soll die Gefahr der Verkeimung auch bei vorhersehbaren, ungünstigen Verhältnissen vermindert werden.

Der Erfolg der Konservierung hängt im wesentlichen von dem Wirkungsspektrum der Konservierungsmittel, der Ausgangskeimzahl, von der Zahl der beim Gebrauch hinzutretenden Keime und dem Wirkungseintritt der Konservierungsmittel ab.

Da schnell und umfassend wirkende Konservierungsmittel gelegentlich zu allergischer Reaktion beim Benutzer führen, wurden Konservierungsmittel vorgeschlagen, die eine gute physiologische Verträglichkeit aufweisen. Hier boten sich aus der Liste der für kosmetische Mittel zugelassenen Konservierungsmittel insbesondere solche an, die auch in Lebensmittel eingesetzt werden können. Oxydierende bzw. reduzierende Mittel können wegen ihrer möglichen Wechselwirkung mit anderen Rezepturbestandteilen nicht verwendet werden.

Aus diesen dem Fachmann bekannten Gründen wurden bereits einige Konservierungsmittel-Kombinationen mit günstigen physiologischen Eigenschaften vorgeschlagen.

In der E-A-34 34 885 werden synergistisch wirkende Kombinationen von Benzoesäure, para-Hydroxybenzoesäureester, Salicylsäure, Sorbinsäure und/oder Ameisensäure sowie deren Derivate und Brenztraubensäure oder deren Salze als antimikrobielle Mittel unter anderen für Kosmetika als Konservierungsmittel beschrieben.

In der E-A-0 365 825 wird eine Kombination aus einem physiologisch verträglichen Salz der Ameisensäure und der Sorbinsäure bzw. deren physiologisch verträglichem Salz und einer oder mehrerer physiologisch verträglicher anorganischer oder gesättigter aliphatischer organischer Säuren beschrieben.

In der E-A-0 287 805 wird eine Kombination aus einem physiologisch verträglichen Salz der Ameisensäure und der Benzoesäure bzw. deren physiologisch verträglichem Salz und einer oder mehrerer physiologisch verträglicher anorganischer oder gesättigter aliphatischer organischer Säuren beschrieben.

Bei Keimzahlprüfungen von Mustern, die aus Gebrauchstesten stammten, sowie Prüfungen mit Beimpfung mit höheren Ausgangskeimzahlen oder mehrfach Verunreinigung wurde gefunden, daß die aus dem vorstehend wiedergegebenen Stand der Technik bekannten Konservierungsmittel und Kombinationen von mehreren Komponenten nicht in allen Fällen zur befriedigenden Konservierung führt.

Die Prüfergebnisse bei einmaliger Kontamination (Impfen) von Haar- und Körperreinigungsmittel als Kulturmedien, die Konservierungsmittel enthalten, entsprechen den in der Praxis vorkommenden Verunreinigungen von in Flaschen oder Tuben enthaltenen kosmetischen Erzeugnissen oder Körperreinigungsmitteln während des Verbrauchs nicht. Mehrfachkontaminationen sind keine Ausnahme, sondern eher die Regel.

Während bei Lebensmitteln oder Arzneimitteln die Verbraucher eine Aufbrauchfrist einmal geöffneter Verpackungen akzeptieren, trifft dies für Kosmetika und Reinigungsmittel nicht zu.

Praxisgerechte Prüfung von in Flaschen und Tuben abgefüllten Haut- und Haarreinigungsmitteln mit den bekannten Konservierungssystemen führten zu Keimbelastungen der Erzeugnisse, die gesundheitliche Gefährdungen nicht mehr ausschließen.
Die an sich naheliegende Erhöhung der Konservierungsmittelkonzentration, um eine sichere Abtötung auch bei höheren Belastungen und Mehrfachkontaminierung sicherzustellen, scheidet bei verkehrsfähigen Produkten wegen der gesetzlichen Mengenbegrenzungen aus.

Denn durch die Verordnung über kosmetische Mittel (BGB1 I S. 1082 vom 26.6.1985 und 22. Änderungsverordnung vom 24.3.1994 (BGB1 I S. 674) ist die Benzoesäure auf ein Maximum von 0,5%, sie Sorbinsäure auf 0,6% und die Ameisensäure auf 0,5% festgesetzt. Das bedeutet, daß die verwendeten Konservierungsmittel in ihrer Konzentration nicht beliebig erhöht werden können.

Aufgabe der Erfindung ist es, ein Mehrkomponentenkonservierungsmittel für Haar- und Körperreinigungsmittel zu schaffen, daß die Nachteile der bekannten Konservierungssysteme vermeidet, den gesetzlichen Bestimmungen genügt und auch bei Erzeugnissen mit niedrigem Tensidgehalt eine ausreichend schnelle Abtötung von durch Verunreinigung eingebrachten Keimen sicherstellt.

Diese Aufgabe wird gelöst durch ein Mehrkomponentenkonservierungsmittel für wässerige Haar- und/oder Körperreinigungsmittel, deren pH-Wert unter 7 beträgt und die von 5-20 Gew.%, bezogen auf Gesamtgewicht, ein oder mehrere Tenside enthalten, das aus einer Kombination von
(A) Ameisensäure und/oder der entsprechenden Menge eines physiologisch verträglichen Salzes von Ameisensäure,
(B) Benzoesäure und/oder der entsprechenden Menge eines physiologisch verträglichen Salzes von Benzoesäure, und
(C) Sorbinsäure und/oder der entsprechenden Menge eines physiologisch verträglichen Salzes von Sorbinsäure besteht.

Die lösung schließt auch ein wäßriges Haar- und/oder Körperreinigungsmittel ein mit einem pH-Wert unter 7 und einem Gehalt von 5-20 Gew.-%, bezogen auf Gesamtgewicht, eines oder mehrerer Tenside, enthaltend ein Mehrkomponentenkonservierungsmittel bestehend aus
(A) Ameisensäure und/oder einem physiologisch verträglichen Salz von Ameisensäure,
(B) Benzoesäure und/oder einem physiologisch verträglichen Salz von Benzoesäure,
(C) Sorbinsäure und/oder einem physiologisch verträglichen Salz von Sorbinsäure,

Völlig überraschend wurde gefunden, daß die Kombination der drei an sich bekannten Konservierungsmittel die Aufgabe löst, obwohl die Bestandteile einzeln oder Zweierkombinationen die Aufgabe nicht lösen. Die mit dem erfindungsgemäßen Mehrkomponentenkonservierungsmittel stabilisierten Haar- und Körperreinigungsmittel weisen vorzugsweise einen pH-Wert von 4,5 bis 5,8 auf.

Das Gewichtsverhältnis der Bestandteile des Mehrkomponentenkonservierungsmittels beträgt vorzugsweise Ameisensäure(salz):Benzoesäure(salz):Sorbinsäure(salz) gleich 1:1:1, es kann jedoch zwischen 1-5:1-5:1-6 betragen.

Die Konzentrationen, bezogen auf Gesamtgewicht des Reinigungsmittels, betragen vorzugsweise

| | |
|---|---|
| 0,1 bis 0,5 Gew.% | Ameisensäure oder der entsprechenden Menge Ameisensäuresalz, |
| 0,1 bis 0,5 Gew.% | Benzoesäure oder der entsprechenden Menge Benzoesäuresalz, |
| 0,1 bis 0,6 Gew.% | Sorbinsäure oder der entsprechenden Menge Sorbinsäuresalz. |

Die entsprechenden Mengen sind die den Säuren äquivalenten Mengen.

Die erfindungsgemäß konservierten wässerigen Haarreinigungsmittel und/oder Körperreinigungsmittel enthalten mindestens ein anionisches, nicht-ionisches oder amphoteres Tensid oder Mischungen dieser Tenside in Mengen von 5-20 Gew.%, bezogen auf Gesamtgewicht.

Beispiele von anionischen Tensiden sind Seifen, die einen hohen Anteil an C₁₀ bis C₁₈ -Fettsäuren aufweisen, Salze von carboxymethylierten Oxäthylaten, Kondensationsprodukte aus Fettsäuren und Aminosäuren, Fettsäure-Eiweißkondensationsprodukte, Akylsulfamidocarbonsäuren, Alkansulfonate, ∝-Olefinsulfonate aus linearen ∝-Olefinen, wie ∝-Dodecen, ∝-Hexadecen, Dinatriumsalze von Sulfobernsteinsäureestern, Alkoxy-, Acyloxy- und Acylaminoalkansulfonate, Alkylsulfate, Salze von Schwefelsäurehalbestern der Alkyl oder Alkylaryloligoglykoläther (Äthersulfate).

Beispiele für nichtionogene Tenside sind ethoxylierte Fettalkohole von C₁₂-C₁₈-Alkoholen, ethoxylierte Fettsäureamide und Fettamine, Polyglycerylether von gesättigten oder ungesättigten Fettalkoholen und Alkylphenolen mit mehr als 8 C-Atomen im Alkylrest und 1-10 Glyceryleinheiten im Molekül, ethoxylierte Sorbitanfettsäureester und Alkylpolyglucoside.

Geeignete amphotere Tenside sind beispielsweise Carboxylderivate von Imidazol, N-Alkylbetaine, Fettsäurealkylamidobetaine, N-Alkylaminobetaine, N-Alkylsulfobetaine, N-Alkylaminopropionate.

Zusätzlich können auch übliche Zusätze, wie sie für derartige kosmetische Mittel verwendet werden, enthalten sein. Übliche Zusätze sind beispielsweise Parfümöle, Perlglanzmittel, Trübungsmittel, Viskositätsregulatoren, Farbstoffe und Komplexbildner. Auch Wirkstoffe wie Kämmhilfen, Antischuppenmittel und Rückfetter können Bestandteil der Formulierung sein.

Bei den üblichen Belastungsprüfungen nach DAB10 erfolgen diese mit definierten Teststämmen verschiedener Keimarten, insbesondere
Escherichia coli ATCC 8739
Pseudomonas aeruginosa ATCC 9027
Staphyloccocus aureus ATCC 6538
Candida albicans ATCC 10231
Aspergillus niger ATCC 16404

Diese Mikroorganismen werden in festgelegter Quantität (10⁵⁻⁶ Keime) dem Produkt zugegeben.

Nach 1, 7, 14, 21 und 28 Tagen werden Proben entnommen und auf Keimzahl untersucht.

| Findet eine Keimzahlminderung bei | |
|---|---|
| P.aeruginosa | nach 14 Tagen um 10³ |
| S.aureus | nach 28 Tagen um 10³ |
| C.albicans | nach 14 Tagen um 10³ |
| A.niger | nach 28 Tagen um 10¹ |

statt, werden die konservierenden Wirkungen als ausreichend angesehen (gemäß DAB 10).

Ergänzend werden vielfach Prüfungen mit Keimen aus dem Produktionsbereich des Herstellers durchgeführt. Hierdurch wird die Produktbelastung durch die Produktionsbedingungen simuliert. Für viele übliche Produktions- und Anwendungsbedingungen hat sich diese Prüfmethode als Grundlage für Entscheidungen über Verkehrsfähigkeit von Haut- und Haarreinigungsmittel bewährt.

Wie bereits erläutert, entsprechen diese Bedingungen nur mit Einschränkungen den Anforderungen in der Praxis.

Die erfindungsgemäße Kombination führt auch bei höheren Anfangskeimzahlen und Mehrfachkontamination (Beimpfen bei Prüfungen) zu der erwünschten keimtötenden Wirkung.

Die nachfolgenden Beispiele sollen dies zeigen, wobei die Bestandteile teilweise mit INCI-Namen (International Nomenclature Cosmetic Ingredient) gekennzeichnet werden.

| Vergleichsbeispiel 1 | |
|---|---|
| | Gew.Tl. |
| Laurylpolyglucosid (Plantaren®1200) | 3,50 |
| Natriumlaurylethersulfat (Texapon®N70) | 11,00 |
| Kokosamidopropylbetain (Tego®Betain F50) | 3,50 |
| Natriumbenzoat | 0,50 |
| Ethylenglycolmonolaurylether (Laureth 2) | 1,00 |
| Citronensäure | 0,35 |
| hydrolysiertes Weizenprotein (Diagluteen®GE20) | 0,75 |
| Parfüm | 0,30 |
| 50% wässeriges KOH | 0,20 |
| Hydroxypropylguar(an) (Jaguar®C162) | 0,10 |
| Dilinoleinsäure | 0,10 |
| 2-Butyloctanol (Isofol®12) | 0,10 |
| 22% wässerige NaCl-Lösung | 1,73 |
| Wasser | 76,97 |

Der pH-Wert der Formulierung beträt 5,0.

| Konservierungsprüfung | | | | | |
|---|---|---|---|---|---|
| Keimart | Staphilococcus aureus | Escherichia coli | Pseudomonas auruginosa | Aspergillus niger | Candida albicans |
| Beimpfte Ausgangs-Keimzahl | 7,55x10⁶ | 2,5x10⁸ | 10⁹ | 9x10⁴ | 4x10⁵ |
| Keimzahl nach | | | | | |
| 1 Tag | <10³ | 4,2x10⁷ | 1,7x10⁶ | 2x10⁵ | 2,5x10⁵ |
| 7 Tagen | <10² | <10² | <10² | <10² | <10² |
| 14 Tagen | <10² | <10² | <10² | <10² | <10² |
| 21 Tagen | <10² | <10² | 10⁹ | <10² | <10² |
| 28 Tagen | <10² | <10² | 10⁹ | <10² | <10² |

Nach 7 Tagen wurden alle Keime auf einen Wert unter 100 reduziert. Nach 21 Tagen ist Pseudomonas aeruginosa auf einen Wert von 10⁹ gestiegen, der im weiteren Testverlauf auch nicht wieder reduziert wurde. Praxisteste mit dem Vergleichsbeispiel 1 mit 20 Testpersonen ergaben nach 3 Wochen Benutzungszeit bei 3 Personen Keimzahlen von über 10⁵.

| Vergleichsbeispiel 2 | |
|---|---|
| | Gew.Tl. |
| Laurylpolyglucosid (Plantaren®1200) | 3,50 |
| Natriumlaurylethersulfat (Texapon®N70) | 11,00 |
| Kokosamidopropylbetain (Tego®Betain F50) | 3,50 |
| Natriumbenzoat | 0,40 |
| Natriumformiat | 0,22 |
| Panthenol (D-Pantenol 75L) | 0,133 |
| Citronensäure | 0,26 |
| Polyethylenglykol(200)stearylether (Steareth 4) | 3,00 |
| Ethylenglycolmonolaurylether (Laureth 2) | 0,33 |
| Dilinoleinsäure | 0,10 |
| Hydroxypropylguar(an) (Jaguar®C162) | 0,10 |
| 2-Butyloctanol (Isofol®12) | 0,10 |
| 22% wässerige NaCl-Lösung | 3,48 |
| Parfüm | 1,00 |
| Wasser | 72,88 |

Der pH-Wert der Formulierung beträgt 5,0.

| Konservierungsprüfung | | | | | |
|---|---|---|---|---|---|
| Keimart | Staphilococcus aureus | Escherichia coli | Pseudomonas auruginosa | Aspergillus niger | Candida albicans |
| Beimpfte Ausgangs-Keimzahl | 7,55x10⁶ | 2,5x10⁸ | 10⁹ | 9x10⁴ | 4x10⁵ |
| Keimzahl nach | | | | | |
| 1 Tag | <10³ | 2,5x10⁸ | 3,9x10⁵ | 6x10⁴ | 4,5x10⁵ |
| 7 Tagen | <10² | 1x10² | <10² | <10² | <10² |
| 14 Tagen | <10² | <10² | <10² | <10² | <10² |
| 21 Tagen | <10² | 5x10⁷ | <10² | <10² | <10² |
| 28 Tagen | <10² | 5x10⁷ | <10² | <10² | <10² |

Nach 14 Tagen sind die Testkeime auf unter 100 Keime reduziert. Es findet jedoch hier ein Wachstum des Escherichia coli auf 5x10⁷ Keime statt.

Praxisteste mit dem Vergleichsbeispiel 2 mit 20 Testpersonen ergaben nach 3 Wochen Benutzungszeit bei 4 Personen Keimzahlen von über 10⁵.

| Vergleichsbeispiel 3 | |
|---|---|
| | Gew.Tl. |
| Laurylpolyglucosid (Plantaren®1200) | 4,00 |
| Natriumlaurylethersulfat (Texapon®N70) | 13,60 |
| Kokosamidopropylbetain (Tego®Betain F50) | 4,00 |
| Ethylenglycolmonolaurylether (Laureth 2) | 0,80 |
| PEG-7-Glycerylkokoat(Cetiol®HE) | 1,20 |
| Kaliumsorbat | 0,20 |
| Natriumformiat | 0,22 |
| Citronensäure | 0,33 |
| 50%iges wässeriges KOH | 0,20 |
| 22% wässerige NaCl-Lösung | 0,80 |
| Parfüm | 1,00 |
| Dilinoleinsäure | 0,10 |
| 2-Hexyldecanol (Isofol® 16) | 0,10 |
| Hydroxypropylguar(an) (Jaguar®C162) | 0,10 |
| Wasser | 73,35 |

Der pH-Wert der Formulierung beträgt 5,1.

| Konservierungsprüfung | | | | | |
|---|---|---|---|---|---|
| Keimart | Staphilococcus aureus | Escherichia coli | Pseudomonas auruginosa | Aspergillus niger | Candida albicans |
| Beimpfte Ausgangs-Keimzahl | 4,8x10⁷ | 3x10⁷ | 3,1x10⁷ | 2x10⁶ | 2x10⁷ |
| Keimzahl nach | | | | | |
| 1 Tag | 2x10⁴ | 1,2x10⁷ | 3,1x10⁷ | 8x10⁵ | 4x10⁶ |
| 7 Tagen | <10³ | <10³ | <10³ | 3x10⁵ | 1x10⁶ |
| 14 Tagen | <10² | 10⁶ | 10⁶ | 3x10³ | 4x10³ |
| 21 Tagen | <10² | 10⁶ | <10⁶ | 5x10² | 6x10⁴ |
| 28 Tagen | <10² | 10⁸ | <10⁸ | <10² | 2x10³ |

Nach 1 und 7 Tagen findet eine Keimreduzierung statt, nach 14 Tagen ist jedoch besonders bei den Bakterien ein starkes Wachstum zu beobachten.

Praxisteste mit dem Vergleichsbeispiel 3 mit 20 Testpersonen ergaben nach 3 Wochen Benutzungszeit bei 3 Personen Keimzahlen von über 10⁵.

### Beispiel 1

| | Gew.Tl. |
|---|---|
| Laurylpolyglucosid (Plantaren®1200) | 4,00 |
| Natriumlaurylethersulfat (Texapon®N70) | 13,60 |
| Kokosamidopropylbetain (Tego®Betain F50) | 4,00 |
| Ethylenglycolmonolaurylether (Laureth 2) | 0,80 |
| PEG-7-Glycerylkokoat(Cetiol®HE) | 1,20 |
| Kaliumsorbat | 0,20 |
| Natriumbenzoat | 0,35 |
| Natriumformiat | 0,22 |
| Citronensäure | 0,50 |
| 50%iges wässeriges KOH | 0,20 |
| Parfüm | 1,00 |
| 2-Hexyldecanol (Isofol® 16) | 0,10 |
| Dilinoleinsäure | 0,10 |
| Hydroxypropylguar(an) (Jaguar®C162) | 0,10 |
| Wasser | 73,65 |

Der pH-Wert der Formulierung beträgt 5,2.

| Konservierungsprüfung | | | | | |
|---|---|---|---|---|---|
| Keimart | Staphilococcus aureus | Escherichia coli | Pseudomonas auruginosa | Aspergillus niger | Candida albicans |
| Beimpfte Ausgangs-Keimzahl | 7,6x10⁷ | 2,8x10¹⁰ | 6x10⁹ | 3x10⁶ | 2,6x10⁷ |
| Keimzahl nach | | | | | |
| 1 Tag | <10⁴ | 1,1x10⁸ | 4,2x10⁵ | 2,4x10⁶ | 2x10⁶ |
| 7 Tagen | <10² | <10² | <10² | <10² | <10² |
| 14 Tagen | <10² | <10² | <10² | <10² | <10² |
| 21 Tagen | <10² | <10² | <10² | <10² | <10² |
| 28 Tagen | <10² | <10² | <10² | <10² | <10² |

Nach 7 Tagen sind die Keime auf unter 100 reduziert. Praxisteste mit dem Beispiel 1 mit 20 Testpersonen ergaben nach 3 Wochen Benutzungszeit jeweils Keimzahlen von 0. Das Beispiel 1 zeigt, daß die Testkeime in einer deutlich kürzeren Zeit abgetötet werden, als in den Beispielen 1-3 und kein nachfolgendes Wachstum zu beobachten ist. Durch den Praxistest wird dieses Ergebnis bestätigt.

## Patentansprüche

1. Mehrkomponentenkonservierungsmittel für wäßrige Haarund Körperreinigungsmittel, deren pH-Wert unter 7 beträgt und die 5-20 Gew.-%, bezogen auf Gesamtgewicht, eines oder mehrerer Tenside enthalten, das aus einer Kombination von
(A) Ameisensäure und/oder einem physiologisch verträglichen Salz von Ameisensäure,
(B) Benzoesäure und/oder einem physiologisch verträglichen Salz von Benzoesäure,
(C) Sorbinsäure und/oder einem physiologisch verträglichen Salz von Sorbinsäure
besteht.

2. Wäßriges Haar- und/oder Körperreinigungsmittel mit einem pH-Wert unter 7 und einem Gehalt von 5-20 Gew.-%, bezogen auf Gesamtgewicht, eines oder mehrerer Tenside, enthaltend ein Mehrkomponentenkonservierungsmittel bestehend aus
(A) Ameisensäure und/oder einem physiologisch verträglichen Salz von Ameisensäure,
(B) Benzoesäure und/oder einem physiologisch verträglichen Salz von Benzoesäure,
(C) Sorbinsäure und/oder einem physiologisch verträglichen Salz von Sorbinsäure.

3. Wäßriges Haar- und/oder Körperreinigungsmittel nach Anspruch 2,
dadurch gekennzeichnet,
daß der pH-Wert von 4,5 bis 5,8 beträgt.

4. Wäßriges Haar- und/oder Körperreinigungsmittel nach Ansprüchen 2 oder 3,
dadurch gekennzeichnet,
daß der Gehalt an Bestandteil (A) von 0,1 % bis 0,5 Gew.-%, bezogen auf Gesamtgewicht, beträgt.

5. Wäßriges Haar- und/oder Körperreinigungsmittel nach Ansprüchen 2 bis 4,
dadurch gekennzeichnet,
daß der Gehalt an Bestandteil (B) von 0,1 % bis 0,5 Gew.-%, bezogen auf Gesamtgewicht, beträgt.

6. Wäßriges Haar- und/oder Körperreinigungsmittel nach Ansprüchen 2 bis 5,
dadurch gekennzeichnet,
daß der Gehalt an Bestandteil (C) von 0,1 % bis 0,6 Gew.-%, bezogen auf Gesamtgewicht, beträgt.

7. Wäßriges Haar- und/oder Körperreinigungsmittel nach Ansprüchen 2 bis 6,
dadurch gekennzeichnet,
daß das Mittel eine Mischung aus anionischen und amphoteren und/oder nichtionischen Tensiden enthält.

## Claims

1. A multi-component preservative composition for aqueous hair and body cleansing compositions having a pH-value below 7 and comprising 5 to 20 wt-% based on the total weight of one or more surfactants whereby the preservative composition consists of a combination of
(A) formic acid and/or a physiologically acceptable salt of formic acid,
(B) benzoic acid and/or a physiologically acceptable salt of benzoic acid,
(C) sorbic acid and/or a physiologically acceptable salt of sorbic acid.

2. An aqueous hair and/or body cleansing composition having a pH-value below 7 and a content of 5 to 20 wt-% based on the total weight of one or more surfactants comprising a multi-component preservative composition consisting of
(A) formic acid and/or a physiologically acceptable salt of formic acid,
(B) benzoic acid and/or a physiologically acceptable salt of benzoic acid,
(C) sorbic acid and/or a physiologically acceptable salt of sorbic acid.

3. An aqueous hair and/or body cleansing composition according to claim 2,
characterized in that it has a pH-value of 4.5 to 5.8.

4. An aqueous hair and/or body cleansing composition according to claims 2 or 3,
characterized in that the content of component (A) is from 0.1 to 0.5 wt-% based on the total weight.

5. An aqueous hair and/or body cleansing composition according to claims 2 to 4,
characterized in that the content of component (B) is from 0.1 to 0.5 wt-% based on the total weight.

6. An aqueous hair and/or body cleansing composition according to claims 2 to 5,
characterized in that the content of component (C) is from 0.1 to 0.6 wt-% based on the total weight.

7. An aqueous hair and/or body cleansing composition according to claims 2 to 6,
characterized in that the composition comprises a mixture of anionic and amphoteric and/or nonionic surfactants.

## Revendications

1. Conservateur à plusieurs composants pour des produits aqueux de nettoyage capillaire et corporel ayant un pH inférieur à 7 et contenant de 5 à 20% en poids, ramené au poids total, d'un ou plusieurs agents tensioactifs, ledit conservateur se composant d'une combinaison :
(A) d'acide formique et/ou d'un sel physiologiquement compatible de l'acide formique,
(B) d'acide benzoïque et/ou d'un sel physiologiquement compatible de l'acide benzoïque,
(C) d'acide sorbique et/ou d'un sel physiologiquement compatible de l'acide sorbique.

2. Produit aqueux de nettoyage capillaire et/ou corporel ayant un pH inférieur à 7 et une teneur de 5 à 20% en poids, ramenée au poids total, d'un ou plusieurs agents tensioactifs, contenant un conservateur à plusieurs composants composé :
(A) d'acide formique et/ou d'un sel physiologiquement compatible de l'acide formique,
(B) d'acide benzoïque et/ou d'un sel physiologiquement compatible de l'acide benzoïque,
(C) d'acide sorbique et/ou d'un sel physiologiquement compatible de l'acide sorbique.

3. Produit aqueux de nettoyage capillaire et/ou corporel selon la revendication 2, caractérisé en ce que le pH est de 4,5 à 5,8.

4. Produit aqueux de nettoyage capillaire et/ou corporel selon l'une des revendications 2 ou 3, caractérisé en ce que la teneur en composant (A) est de 0,1 à 0,5 % en poids, ramenée au poids total.

5. Produit aqueux de nettoyage capillaire et/ou corporel selon les revendications 2 à 4, caractérisé en ce que la teneur en composant (B) est de 0,1 à 0,5 % en poids, ramenée au poids total.

6. Produit aqueux de nettoyage capillaire et/ou corporel selon les revendications 2 à 5, caractérisé en ce que la teneur en composant (C) est de 0,1 à 0,6 % en poids, ramenée au poids total.

7. Produit aqueux de nettoyage capillaire et/ou corporel selon les revendications 2 à 6, caractérisé en ce que le produit contient un mélange d'agents tensioactifs anioniques et amphotères et/ou non ioniques.
